# EUROPÄISCHE PATENTSCHRIFT

(11) **EP 4 255 511 B1**
(45) Veröffentlichungstag und Bekanntmachung des Hinweises auf die Patenterteilung: **25.06.2025**
(21) Anmeldenummer: 21815153.8
(22) Anmeldetag: 16.11.2021
(51) Int. Cl.: A61L 2/14, D06M 10/02, H05H 1/24, H05H 1/46, H05H 1/48

(54) **PLASMA-VORRICHTUNG**
PLASMA DEVICE
DISPOSITIF À PLASMA

(30) Priorität: 01.12.2020 DE 102020215112
(43) Veröffentlichungstag der Anmeldung: 11.10.2023
(73) Patentinhaber: BSH Hausgeräte GmbH, 81739 München (DE)
(72) Erfinder: HASSFURTER, Stefan, 96126 Maroldsweisach (DE); WENDE, Tobias, 83209 Prien (DE); GEIS, Julius, 97688 Bad Kissingen (DE)
(86) Internationale Anmeldenummer: PCT/EP2021/081796
(87) Internationale Veröffentlichungsnummer: WO 2022/117329

(56) Entgegenhaltungen:
- DE-A1- 102018 213 143
- DE-A1- 102018 213 144
- DE-U1- 202018 006 360

## Beschreibung

Die vorliegende Erfindung betrifft eine Plasma-Vorrichtung zum Behandeln von Oberflächen bei Textilien, gemäß Anspruch 1, sowie eine Plasma-Vorrichtung zum Behandeln von Oberflächen gemäß Anspruch 14. Weitere bevorzugte erfinderische Ausführungen sind in den abhängigen Ansprüchen definiert.

Aus der DE 10 2011 100 751 A1 ist eine gattungsgemäße Plasma-Vorrichtung zur Inaktivierung vorzugsweise geruchsrelevanter Moleküle und damit zum Auffrischen von Oberflächen, beispielsweise bei Textilien, bekannt. Die Plasma-Vorrichtung weist ein Gehäuse sowie eine zugeordnete Plasmaquelle und mindestens ein Abstandsmittel auf, welches dafür sorgt, dass zwischen der Plasmaquelle und einer zu behandelnden Oberfläche ein vordefinierter Abstand eingehalten wird, bei welchem an der zu behandelnden Oberfläche angelagerte Moleküle durch Elektronen des Plasmas inaktiviert werden können.

Plasma-Vorrichtungen mit einem runden Gehäuse sind z.B. aus der DE 10 2018 213144 A1, der DE 10 2018 213143 A1 und der DE 20 2018 006360 U1 bekannt.

Eine schnelle Reinigung von Oberflächen, beispielsweise Textilien, ist jedoch nicht immer mittels Waschen oder Reinigen möglich, so dass hierfür beispielsweise Plasma-Vorrichtungen eingesetzt werden, mittels welchen insbesondere Bakterien, Keime, Viren, Sporen, Pilze und Geruchsmoleküle inaktiviert werden können.

Aus der CN 205 814 739 U ist eine tragbare Sterilisations- und Desinfektionsmaschine bekannt, mit einem Gehäuse, einer Energiespeichervorrichtung, die in dem Gehäuse angeordnet ist, sowie mit einem Transformator. Eine Ausgangsspannung der Energiespeichervorrichtung wird hierbei verstärkt und über den Transformator so konfiguriert, dass mit ihr Ozon sowie negative Ionen durch Hochspannungsentladung unter der von dem Transformator ausgegebenen Verstärkungsspannung erzeugt werden können.

Aus der CN 108 771 767 A ist eine Reinigungsvorrichtung zum Reinigen von Kleidern bekannt, mit einem Gehäuse, in dem ein Aufnahmeraum zur Aufnahme eines Gebläses vorgesehen ist. Ebenfalls in dem Aufnahmeraum vorgesehen ist ein Abscheider, der einen Lufteinlassbereich von einem Reinigungsbereich trennt. Die genannten Bereiche sind dabei im Betriebszustand vertikal übereinander angeordnet. Mit der Reinigungsvorrichtung lassen sich die zu behandelnden Kleidungsstücke insbesondere von Zigarettenraum befreien bzw. reinigen.

Nachteilig bei den aus dem Stand der Technik bekannten Geräten ist oftmals, dass diese, insbesondere bei längerer Anwendung schwer und unkomfortabel in einer Hand einer Benutzerin bzw. eines Benutzers liegen und dadurch den Bedienkomfort beeinträchtigen.

Die vorliegende Erfindung beschäftigt sich daher mit dem Problem, für eine Plasma-Vorrichtung der gattungsgemäßen Art eine verbesserte oder zumindest eine alternative Ausführungsform anzugeben, die insbesondere die aus dem Stand der Technik bekannten Nachteile überwindet.

Dieses Problem wird erfindungsgemäß durch den Gegenstand des unabhängigen Anspruchs 1 gelöst. Vorteilhafte Ausführungsformen sind Gegenstand der abhängigen Ansprüche.

Die vorliegende Erfindung beruht auf dem allgemeinen Gedanken, eine einhändig zu bedienende Plasma-Vorrichtung zum Behandeln von Oberflächen, insbesondere zum Auffrischen von Textilien, die insbesondere als Textilienbehandlungs-Plasma-Vorrichtung ausgeführt sein kann, derart auszubilden, dass diese langfristig einhändig komfortabel benutzbar ist, indem ein bestimmtes Verhältnis eines Massenschwerpunktes M zu einem geometrischen Schwerpunkt G, das heißt zu einem Volumenschwerpunkt, vorgegeben wird. Die erfindungsgemäße Plasma-Vorrichtung weist dabei ein längliches und einhändig bedienbares Gehäuse auf, welches von einer Hand einer Benutzerin bzw. eines Benutzers leicht und formschlüssig umgriffen werden kann. In dem Gehäuse ist eine Plasmaquelle angeordnet, die eine in Längsrichtung des Gehäuses verlaufende Plateaufläche mit zumindest einer Elektrode, beispielsweise einer leicht erhabenen Elektrode mit einem diese umgebenden Keramiksubstrat, aufweist, über die Plasma erzeugbar und auf die behandelnde Oberfläche, beispielsweise auf die aufzufrischende Textilie, ausbringbar ist. Erfindungsgemäß weisen nun ein Massenschwerpunkt M sowie ein geometrischer Schwerpunkt G einen Abstand A von weniger als 20 mm auf. Durch den erfindungsgemäß eingeschränkten Abstand A zwischen Massenschwerpunkt M und geometrischem Schwerpunkt G, das heißt zwischen Massenschwerpunkt M und Volumenschwerpunkt, lässt sich die handgeführte Plasma-Vorrichtung leicht bewegen, drehen, heben und neigen, da nur äußerst geringe Momente bei einem Neigen, Drehen, etc. auftreten. Durch die erfindungsgemäße Anordnung des Massenschwerpunkts M lässt sich zudem eine Drückbewegung einer Hand der Benutzerin bzw. des Benutzers in Aktivierungsrichtung unterstützen. Das längliche Gehäuse besitzt dabei eine im Querschnitt im Wesentlichen ovale Gestalt und ist damit optimal an eine Handinnenfläche angepasst. Um dabei sowohl eine Bedienung für einen Rechtshänder als auch für einen Linkshänder gleichermaßen komfortabel gestalten zu können, kann die erfindungsgemäße Plasma-Vorrichtung bzw. deren Gehäuse bezüglich einer Längsmittelebene bzw. Quermittelebene symmetrisch ausgebildet sein. Durch den erfindungsgemäß vorgegebenen maximalen Abstand A zwischen Massenschwerpunkt M und geometrischen Schwerpunkt G von weniger als 20 mm kann darüber hinaus eine äußerst angenehm empfundene Ergonomie und damit eine leichte und komfortable Bedienbarkeit der Plasma-Vorrichtung geschaffen werden.

Neben den drei Aggregatzuständen fest, flüssig oder gasförmig, wird Plasma als vierter Aggregatzustand bezeichnet. Wird einem Gas oder Gasgemisch hinreichend viel Energie, beispielsweise in Form von elektrischer Energie, zugefügt, so werden einige Atome des Gases ionisiert, d. h. Elektronen werden aus der Atomhülle entfernt und bewegen sich als freie Teilchen, sodass ein positiv geladenes Atom zurückbleibt. Wenn ein Gas aus einem ausreichend hohen Anteil an freien Ionen und Elektronen besteht, so bezeichnet man den Aggregatzustand als Plasma. Plasma ist also Materie, dessen Bestandteile teilweise geladene Komponenten, Ionen und Elektronen sind, die sich als freie Ladungsträger bewegen.

Nichtthermisches Plasma, das auch als kaltes Plasma bezeichnet wird, kann gezielt zur Beseitigung von Gerüchen und bestimmten Kohlenwasserstoffen eingesetzt werden. Weiterhin finden nichtthermische Plasmen Anwendung in der Medizintechnik, zum Beispiel bei der Behandlung von schlecht oder nicht heilenden Wunden mit Hilfe eines sogenannten Plasmastifts, indem auf die antimikrobielle Wirkung von "kaltem Plasma" zurückgegriffen wird.

Die Ursachen der antibakteriellen Wirkung eines Plasmas liegen in Hitze, Austrocknung, Scherspannung, UV-Strahlung, freie Radikale und Ladungen. Bei kalten Plasmen, wie sie in der erfindungsgemäßen Plasma-Vorrichtung verwendet werden, spielt die Hitze eine untergeordnete Rolle, da diese Plasmen bei Raumtemperatur betrieben werden. In solchen Niederdruckplasmen entstehen besonders reaktive Partikel, wie beispielsweise verschiedene Sauerstoff- oder Stickstoffspezies, die eine ausreichend hohe Lebensdauer aufweisen, um bei einer indirekten Exposition organische Verbindungen zu schädigen. Zu diesen Partikeln zählen unter anderem atomarer Sauerstoff, Superoxidradikale, Ozon, Hydroxylradikale, Stickstoffmonooxid und Stickstoffdioxid. Diese Partikel zeigen eine zerstörerische Wirkung auf unterschiedlichste Geruchskomponenten wie auch Zellkomponenten. Werden Geruchskomponenten, welche meist aus Kohlenstoffverbindungen bestehen, wie auch Zellwände von Bakterien, Keimen, Viren, Pilzen oder anderen vergleichbaren Mikroorganismen dem Plasma direkt ausgesetzt, so laden sich diese aufgrund des Beschusses mit den im Plasma vorhandenen Elektronen negativ auf. Aufgrund der elektrostatischen Abstoßung führt dies zu mechanischen Spannungen bis hin zur Überschreitung der Zugfestigkeit und der Zerstörung von dem Geruchsmolekül, bzw. der Zellwand. Aber nicht nur mechanische Verspannungen aufgrund der Ladung können die Zellwände zerstören, sondern auch die Störung des Ladungsgleichgewichts der Geruchsmoleküle, bzw. der Zellwand durch verschiedene, weitere elektrostatische Wechselwirkungen und der Elektrolyse, z. B. durch Änderung der Permeabilität der Zellwände. Ein Mechanismus zur Inaktivierung von Mikroorganismen ergibt sich auch aus den sehr energiereichen Ionen. Mittels einer Hochfrequenz kann das Plasma erzeugt werden. Niederdruckplasmen sind daher besonders gut zur Inaktivierung von Gerüchen an textilem Gewebe oder von haushaltsüblichen Oberflächen oder dergleichen geeignet, um eine Geruchsinaktivierung zu erreichen.

Die zu behandelnden Oberflächen können dabei textile Materialien mit natürlichen, pflanzlichen oder tierischen Fasern, wie z. B. Baum-, Schafwolle, Seide, Leinen, Filz oder künstlichen Fasern, wie z.B. Nylon, sein. Weiter sollen unter dem Begriff "Oberfläche" auch Materialien und Gegenstände aus Keramik, Kunststoff, Federn, Leder, Glas, Holz, oder Metall verstanden werden können.

Die mit der erfindungsgemäßen Plasma-Vorrichtung neutralisierbaren geruchsrelevanten Moleküle können Buttersäure, Schweiß, festgesetzter Zigarettenrauch oder generell Gerüche, die als unangenehm empfundenen werden können, sein. Die Plasma-Vorrichtung beruht darauf, dass die Gerüche aktiv beseitigt werden und nicht unangenehme Gerüche, z. B. mittels Parfüm, überdeckt werden. Mit der erfindungsgemäßen Plasma-Vorrichtung können auch nicht-geruchsrelevante Moleküle, wie beispielsweise Allergene, Proteinmoleküle, Prionen, u. ä, zerstört werden.

Bei einer vorteilhaften Weiterbildung der erfindungsgemäßen Lösung weisen der Massenschwerpunkt M und der geometrische Schwerpunkt G einen Abstand A von weniger als 10 mm, insbesondere einen Abstand A von weniger als 5 mm, auf. Hierdurch ist es möglich, den Massenschwerpunkt M sowie den geometrischen Schwerpunkt G zunehmend aneinander zu rücken, wodurch zunehmend weniger Kräfte zum Heben, Drehen und Neigen erforderlich sind und dadurch eine zunehmend komfortablere Benutzung der erfindungsgemäßen Plasma-Vorrichtung gegeben ist. Den Idealfall stellt dabei eine erfindungsgemäße Plasma-Vorrichtung dar, bei welcher der Massenschwerpunkt M sowie der geometrische Schwerpunkt G identisch sind. Hierdurch lässt sich ein besonders ergonomisches und angenehmes Benutzen der Plasma-Vorrichtung sicherstellen.

Zweckmäßig schneidet eine geometrische Längsachse der Plasma-Vorrichtung eine erste Kreisfläche mit einem Radius R₁ ≤ 5 mm, wobei der Massenschwerpunkt M einen Mittelpunkt dieser ersten Kreisfläche bildet. Hierdurch kann gewährleistet werden, dass selbst bei einem außermittigen Andrücken der Plasma-Vorrichtung auf die zu behandelnde Oberfläche eine zuverlässige Bewegung der Plasma-Vorrichtung und Aktivierung der Plasmaquelle gewährleistet werden kann. Bei einer derartigen Ausführungsform kann somit gewährleistet werden, dass auch bei unterschiedlichsten Aufsatz- bzw. Andrückwinkeln eine zuverlässige Aktivierung der Plasmaquelle und damit eine zuverlässige Behandlung, insbesondere Auffrischung, von zu behandelnden Oberflächen, insbesondere Textilien, erfolgen kann.

Bei einer weiteren vorteilhaften Ausführungsform der erfindungsgemäßen Lösung schneidet eine geometrische Querachse der Plasma-Vorrichtung eine zweite Kreisfläche mit einem Radius R₂ ≤ 5 mm, wobei der Massenschwerpunkt M einen Mittelpunkt dieser zweiten Kreisfläche bildet. Zusätzlich oder alternativ kann vorgesehen sein, dass eine geometrische Hochachse der Plasma-Vorrichtung eine dritte Kreisfläche mit einem Radius R₃ ≤ 5 mm schneidet, wobei der Massenschwerpunkt M einen Mittelpunkt der dritten Kreisfläche bildet. Auch bei einer derartigen Begrenzung des Radius der dritten bzw. zweiten Kreisfläche um eine geometrische Hochachse bzw. geometrische Querachse kann gewährleistet werden, dass bei einem außermittigen Drücken der Plasma-Vorrichtung auf die zu behandelnde Oberfläche, beispielsweise auf die zu behandelnde Textilie, die Plasmaquelle zuverlässig aktiviert werden kann. Im Idealfall schneidet dabei die Längsachse, die Querachse und die Hochachse der Plasma-Vorrichtung eine Kugel mit einem Radius von R_{K} ≤ 5 mm, wobei der Massenschwerpunkt M der Plasma-Vorrichtung den Mittelpunkt der Kugel darstellt. Hierdurch ist es für einen Benutzer bzw. eine Benutzerin möglich, die handgeführte Plasma-Vorrichtung kraftarm zu bewegen, insbesondere zu neigen, zu gieren oder zu rollen. Konkret bedeutet dies, dass bei einer Verlagerung der Plasma-Vorrichtung im Raum nur geringe aufzubringende Drehmomente erforderlich sind, so dass die erfindungsgemäße Plasma-Vorrichtung leicht auch an schwierig zugänglichen Stellen der zu behandelnden Oberflächen, beispielsweise der zu behandelnden Textilien, aber auch unter Achseln bei Menschen, geführt werden kann.

Zweckmäßig ist die Plasma-Vorrichtung um zumindest die Längsachse, die Querachse, oder die Hochachse symmetrisch ausgebildet. Dies bietet den großen Vorteil, dass die Plasma-Vorrichtung über ihr Gehäuse einfach gegriffen werden kann und leicht hinsichtlich der Handhabung, insbesondere auch hinsichtlich einer Bewegung ist.

Zweckmäßig ist ein Schalter zum Ein- und Ausschalten der erfindungsgemäßen Plasma-Vorrichtung vorgesehen, wobei der Massenschwerpunkt M und der Schalter versetzt zum geometrischen Schwerpunkt angeordnet sind. Durch den erfindungsgemäß angeordneten Massenschwerpunkt M wird einem Benutzer bzw. einer Benutzerin ein intuitives Ergreifen der Plasma-Vorrichtung erleichtert, wobei der Massenschwerpunkt M vorzugsweise in einer Handfläche liegt und der Schalter leicht mit einem Zeigefinger zu bedienen ist. Darüber hinaus ermöglicht eine derartige Anordnung des Massenschwerpunktes M und des diametral gegenüberliegenden Schalters eine Bedienung der erfindungsgemäßen Plasma-Vorrichtung sowohl von Links- als auch von Rechtshändern sowie insgesamt eine leichte und komfortable einhändige Bedienung/Benutzung.

Zweckmäßig ist die Plasmaquelle federbeaufschlagt und steht in einem Nichtgebrauchszustand der Plasma-Vorrichtung über das Gehäuse derselben über. Durch eine Federbeaufschlagung der Plasmaquelle ist es möglich, die Plateaufläche beim Reinigungsvorgang dicht auf der Oberfläche zu führen, wodurch insbesondere eine übermäßige Ozonproduktion durch die Plasmaquelle verhindert werden kann. Die federbeaufschlagte Plateaufläche bewirkt auch ein Abschirmen des ausgesandten Plasmas vom Luftsauerstoff, so dass zwar genügend Sauerstoff zur Ozonproduktion zur Verfügung steht, die zuvor genannte übermäßige Ozonproduktion mit den damit verbundenen Nachteilen jedoch nicht stattfindet. Die Nachteile einer übermäßigen Ozonproduktion liegen insbesondere bei einer längeren Einwirkung auf die zu behandelnde Oberfläche, beispielsweise eine Textilie, in einer Ver- bzw. Entfärbung derselben sowie in der Gefahr von Atemwegsreizungen.

Weitere wichtige Merkmale und Vorteile der Erfindung ergeben sich aus den Unteransprüchen, aus den Zeichnungen und aus der zugehörigen Figurenbeschreibung anhand der Zeichnungen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Bevorzugte Ausführungsbeispiele der Erfindung sind in den Zeichnungen dargestellt und werden in der nachfolgenden Beschreibung näher erläutert, wobei sich gleiche Bezugszeichen auf gleiche oder ähnliche oder funktional gleiche Komponenten beziehen.

Dabei zeigen, jeweils schematisch,
- Fig. 1: eine Längsschnittdarstellung durch eine erfindungsgemäße Plasma-Vorrichtung,
- Fig. 2: eine Ansicht von oben auf die erfindungsgemäße Plasma-Vorrichtung,
- Fig. 3: eine Querschnittsdarstellung durch die Plasma-Vorrichtung in einer Hand einer Benutzerin bzw. eines Benutzers in einer Nichtgebrauchsstellung,
- Fig. 4: eine Darstellung wie in Fig. 3, jedoch in einer Gebrauchsstellung,
- Fig. 5: eine Ansicht von oben auf eine erfindungsgemäße Plasma-Vorrichtung während der Benutzung.

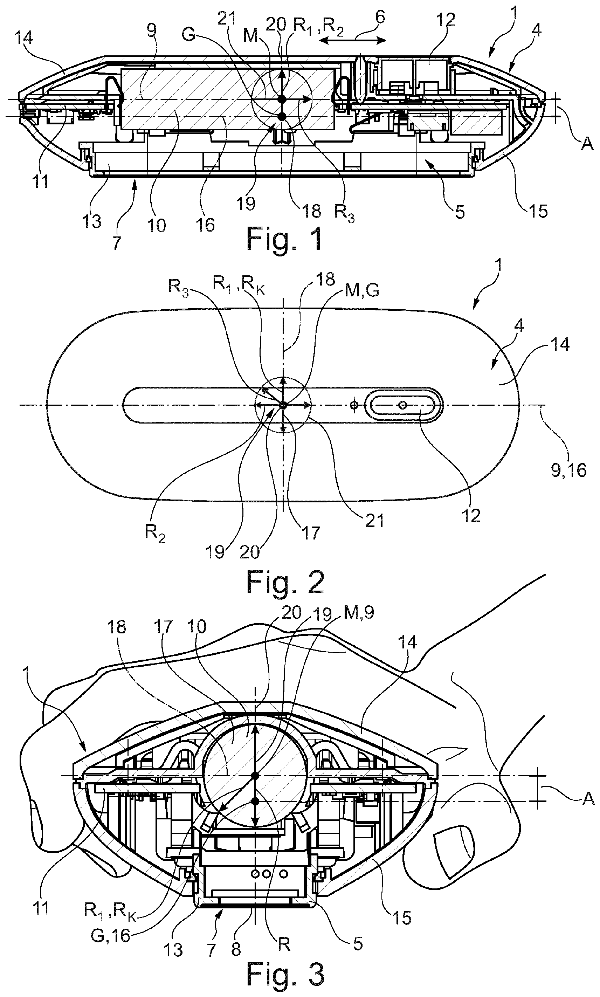

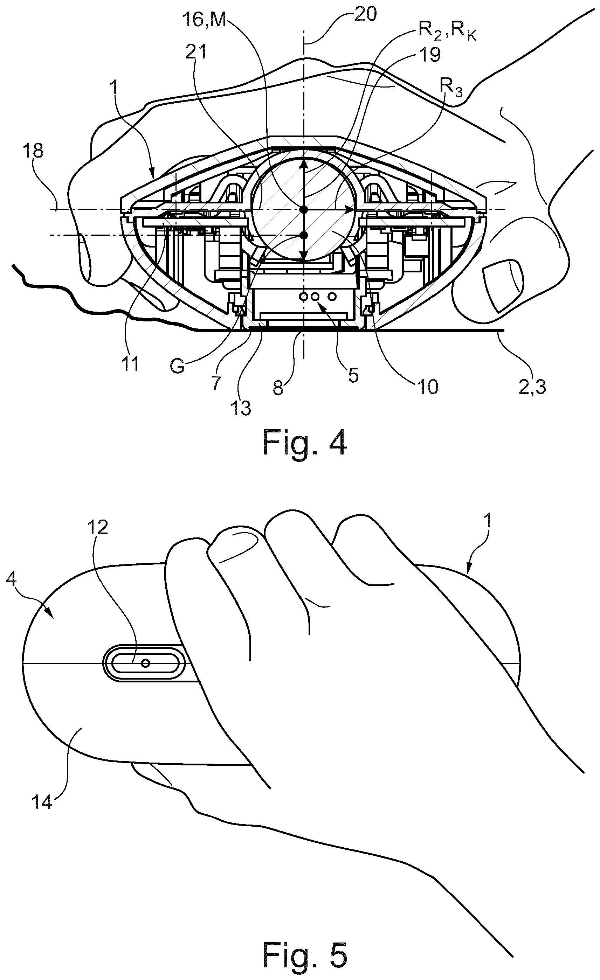

Entsprechend den Fig. 1 bis 5, weist eine erfindungsgemäße Plasma-Vorrichtung 1 zum Behandeln von Oberflächen 2, insbesondere zum Behandeln von Textilien 3 (vgl. Fig. 4) ein längliches und im Querschnitt ovalartiges Gehäuse 4 auf, in welchem eine Plasmaquelle 5 angeordnet ist, die eine in Längsrichtung 6 des Gehäuses 4 verlaufende Plateaufläche 7 mit zumindest einer Elektrode 8 aufweist, über die Plasma auf die zu behandelnde Oberfläche 2 ausbringbar ist. Um nun eine möglichst komfortable und zudem leichte Bedienung bzw. Benutzung der erfindungsgemäßen Plasma-Vorrichtung 1 zu ermöglichen, ist vorgesehen, dass ein Massenschwerpunkt M und ein geometrischer Schwerpunkt G einen Abstand A von weniger als 20 mm aufweisen. Ganz bevorzugt weisen der Massenschwerpunkt M und der geometrische Schwerpunkt G einen Abstand A von weniger als 10 mm, insbesondere einen Abstand A von weniger als 5 mm auf.

Unter dem Massenschwerpunkt M wird der Punkt eines Körpers, hier der Plasma-Vorrichtung 1, verstanden, in dem die gesamte Masse vereinigt gedacht werden kann. Bei Unterstützung des Körpers im Massenschwerpunkt M bleibt der Körper im Gleichgewicht. Der geometrische Schwerpunkt G entspricht dabei dem Massenschwerpunkt eines Körpers, der aus homogenem Material besteht, also überall die gleiche Dichte hat. Unterschiedliche Anordnungen einzelner Komponenten, wie in der vorliegenden Plasma-Vorrichtung 1 dargestellt, aber auch unterschiedliche Dichteverhältnisse, können dazu führen, dass der Massenschwerpunkt M und der geometrische Schwerpunkt G oft auch Volumenschwerpunkt genannt, nicht identisch sind. Um jedoch eine möglichst komfortable Handhabung der Plasma-Vorrichtung 1 gewährleisten zu können, wird angestrebt, den Massenschwerpunkt M und den geometrischen Schwerpunkt G nach Möglichkeit so eng wie möglich beieinander anzuordnen, wobei im Idealfall der geometrische Schwerpunkt G und der Massenschwerpunkt M identisch sind.

Der Massenschwerpunkt M der Plasma-Vorrichtung 1 soll darüber hinaus möglichst mittig der Plasma-Vorrichtung 1 in einer Drehachse eines Unterarms einer Benutzerin bzw. eines Benutzers liegen. Eine Schwerachse 9 der Plasma-Vorrichtung 1 sollte dabei möglichst nahe an einer Handinnenfläche liegen und außerdem bei gestrecktem Arm senkrecht zur Drehachse des Unterarms sein.

Um den geometrischen Schwerpunkt G und den Massenschwerpunkt M mit möglichst kleinem Abstand A zueinander anordnen zu können, müssen einzelne Komponenten der Plasma-Vorrichtung 1, wie beispielsweise ein Energiespeicher 10, eine Hauptplatine 11, die Plasmaquelle 5, ein Schalter 12 sowie ein Halterahmen 13 für die Plasmaquelle 5 entsprechend angeordnet bzw. hinsichtlich ihres Gewichts angepasst werden.

Das Gehäuse 4 weist üblicherweise ein Gehäuseoberteil 14 sowie ein Gehäuseunterteil 15 auf, welche beispielsweise über eine Clipverbindung miteinander verbunden sind. Im Gehäuseunterteil 15 ist bodenseitig eine Öffnung, durch welche die Plasmaquelle 5 aus dem Gehäuse 4 nach außen ragt.

Durch ein möglichst dichtes Anordnen des Massenschwerpunkts M und des geometrischen Schwerpunkts G kann insbesondere auch ein Verkippen einer beweglichen Plasmaquelle 5 verhindert werden, sogar, wenn die Plasma-Vorrichtung 1 außermittig gedrückt wird. Durch einen vergleichsweise kleinen Abstand A zwischen Massenschwerpunkt M und geometrischem Schwerpunkt G kann auch eine leichte Handhabung der Plasma-Vorrichtung 1 hinsichtlich einer Bewegung auf der zu behandelnden Oberfläche 2 erreicht werden, wodurch ein Gebrauch der Plasma-Vorrichtung 1 mit geringem Kraftaufwand möglich sind. Zudem kann ein Verdrehen, Neigen, Kippen, Rollen, etc. der Plasma-Vorrichtung 1 ohne große aufzubringende Drehmomente erfolgen, so dass diese auch an unzugänglichen Stellen, beispielsweise am Körper unter der Achsel, einfach geführt werden können.

Die Plasmaquelle 5 kann darüber hinaus federbeaufschlagt sein und in einem Nichtgebrauchszustand (vgl. Fig. 3) über das Gehäuse 4 nach unten überstehen und in einem Gebrauchszustand (vgl. Fig. 4) zumindest teilweise in das Gehäuse 5 eingedrückt sein und dadurch mit der Plateaufläche 7 flächig auf der zu behandelnden Oberfläche 2 aufliegen. Hierdurch kann auch bei einem Bewegen der Plasma-Vorrichtung 1 eine gleichmäßige Oberflächenberührung sichergestellt werden, wodurch das zur Behandlung der Oberfläche 2 von der Plasmaquelle 5 ausgesandte Plasma nicht mit der Umgebungsluft in Berührung kommt und dadurch auch weniger Ozon erzeugt wird, welches bei einer langfristigen Einwirkung auf die zu behandelnde Oberfläche 2, beispielsweise einer Textilie 3, zu einer Ver- bzw. Entfärbung derselben führen könnte. Auch kann hierdurch die Gefahr einer Atemwegsreizung reduziert werden.

Bei einer vorteilhaften Weiterbildung der erfindungsgemäßen Lösung schneidet eine geometrische Längsachse 16 eine erste Kreisfläche 17 mit einem Radius R₁ ≤ 5 mm, wobei der Massenschwerpunkt M einen Mittelpunkt der ersten Kreisfläche 17 bildet. Die erste Kreisfläche 17 verläuft dabei gemäß der Fig. 1 orthogonal zur Bildebene und gemäß der Fig. 3 in der Bildebene. Gemäß der Fig. 1 verläuft dabei eine Schwerachse 9 ebenso wie die geometrische Längsachse 16 in der Bildebene horizontal, wobei der Massenschwerpunkt M auf der Schwerachse 9 liegt, während der geometrische Schwerpunkt G auf der geometrischen Längsachse 16 liegt. In Fig. 2 sind die geometrische Längsachse 16 und die Schwerachse 9 in Bildebene hintereinander, das heißt deckungsgleich angeordnet. In Fig. 3 verlaufen sowohl die Schwerachse 9 als auch die geometrische Längsachse 16 orthogonal zur Bildebene, während eine geometrische Querachse 18 und die geometrische Hochachse 20 in Bildebene verlaufen.

Betrachtet man die Fig. 1 und 4 weiter so kann man erkennen, dass die geometrische Querachse 18 der Plasma-Vorrichtung 1 eine zweite Kreisfläche 19 mit einem Radius R₂ ≤ 5 mm schneidet, wobei der Massenschwerpunkt M einen Mittelpunkt der zweiten Kreisfläche 19 darstellt. Die zweite Kreisfläche 19 verläuft dabei gemäß der Fig. 4 senkrecht zur Bildebene und liegt in Fig. 1 in der Bildebene.

Bei einer weiteren vorteilhaften Ausführungsform der erfindungsgemäßen Lösung schneidet eine geometrische Hochachse 20, auf welcher der geometrische Schwerpunkt G liegt, eine dritte Kreisfläche 21 mit einem Radius R₃ ≤ 5 mm, wobei der Massenschwerpunkt M einen Mittelpunkt der dritten Kreisfläche 21 darstellt. Die dritte Kreisfläche 21 verläuft dabei gemäß den Fig. 1, 3 und 4 horizontal in der Bildebene und gemäß Fig. 2 in der Bildebene.

Bei einer besonders bevorzugten Ausführungsform der erfindungsgemäßen Plasma-Vorrichtung 1 können die geometrische Längsachse 16, die geometrische Querachse 18 und die geometrische Hochachse 20 eine Kugel mit einem Radius R_{K} < 5 mm schneiden, wobei der Massenschwerpunkt M einen Mittelpunkt der Kugel bildet. Eine derartige Kugel würde dabei aus den einzelnen Kreisflächen 17, 19 und 21 aufgespannt werden.

Die möglichst enge Anordnung bzw. identische Anordnung von Masseschwerpunkt und geometrischem Schwerpunkt G ermöglicht ein vergleichsweise einfaches Verdrehen, Rollen, Nicken, Neigen, etc. der Plasma-Vorrichtung 1 ohne größere Drehmomente, wodurch eine Handhabung und Benutzung der erfindungsgemäßen Plasma-Vorrichtung 1 durch eine Benutzerin bzw. einen Benutzer komfortabel gestaltet werden kann. Die Plasma-Vorrichtung 1 kann darüber hinaus um zumindest die geometrische Längsachse 16, die geometrische Querachse 18 bzw. die geometrische Hochachse 20 symmetrisch ausgebildet sein, insbesondere rotationssymmetrisch, wodurch die Plasma-Vorrichtung 1 haptisch leicht greifbar ist. auch kann hierdurch ein einfaches Handhaben derselben erreicht werden.

Zudem kann vorgesehen sein, dass der Schalter 12 zum Ein- und Ausschalten der Plasma-Vorrichtung 1 und der Massenschwerpunkt M versetzt zum geometrischen Schwerpunkt G angeordnet sind. Der Schalter 12 kann beispielsweise zum Schwerpunkt M, S sein. Der versetzt angeordnete Schalter 12 kann ein leichtes Aktivieren bzw. Deaktivieren der Plasma-Vorrichtung 1 durch einen Zeigefinger des Benutzers bzw. der Benutzerin ermöglichen, wodurch eine intuitive Benutzung ermöglicht wird.

Alles in allem kann mit der erfindungsgemäßen Plasma-Vorrichtung 1 und der vorzugsweise identischen Anordnung des geometrischen Schwerpunkts G und des Massenschwerpunkts M eine vergleichsweise einfache und kraftarme Benutzung derselben ermöglicht werden, wodurch eine deutliche Komfortsteigerung hinsichtlich der Benutzung erreicht werden kann.

Gemäß den Fig. 1, 3 und 4 sind die geometrischen Längsachsen 16 sowie die geometrische Querachse 18 mit jeweils Abstand A versetzt zum Massenschwerpunkt M angeordnet, wobei diese selbstverständlich auch durch den Massenschwerpunkt M gehen können.

### Bezugszeichenliste

- 1: Plasma-Vorrichtung
- 2: Oberfläche
- 3: Textil
- 4: Gehäuse
- 5: Plasmaquelle
- 6: Längsrichtung
- 7: Plateaufläche
- 8: Elektrode
- 9: Schwerachse
- 10: Energiespeicher
- 11: Hauptplatine
- 12: Schalter
- 13: Halterahmen
- 14: Gehäuseoberteil
- 15: Gehäuseunterteil
- 16: geometrische Längsachse
- 17: erste Kreisfläche
- 18: geometrische Querachse
- 19: zweite Kreisfläche
- 20: geometrische Hochachse
- 21: dritte Kreisfläche

## Patentansprüche

1. Plasma-Vorrichtung (1) zum Behandeln von Oberflächen (2) bei Textilien (3), wobei die Plasma-Vorrichtung (1) ein längliches und einhändig bedienbares Gehäuse (4) aufweist, in welchem eine Plasmaquelle (5) angeordnet ist, die eine in Längsrichtung (6) des Gehäuses (4) verlaufende Plateaufläche (7) mit zumindest einer Elektrode (8) aufweist, über die Plasma erzeugbar und auf die zu behandelnde Oberfläche (2) ausbringbar ist, wobei ein Massenschwerpunkt M der Plasma-Vorrichtung (1) und ein geometrischer Schwerpunkt G der Plasma-Vorrichtung (1) einen Abstand A von weniger als 20 mm aufweisen.

2. Plasma-Vorrichtung nach Anspruch 1, wobei die Plasma-Vorrichtung ein im Querschnitt ovalartiges Gehäuse (4) aufweist.

3. Plasma-Vorrichtung nach Anspruch 2, wobei der ovalartige Querschnitt des Gehäuses (4) derart an eine Handinnenfläche angepasst ist, dass das Gehäuse (4) von einer Hand einer Benutzerin bzw. eines Benutzers formschlüssig umgriffen werden kann.

4. Plasma-Vorrichtung nach einem der vorhergehenden Ansprüche, wobei ein Halterahmen (13) für die Plasmaquelle (5) entsprechend angeordnet und/oder hinsichtlich seines Gewichts angepasst ist.

5. Plasma-Vorrichtung nach einem der vorhergehenden Ansprüche, wobei der Massenschwerpunkt M und der geometrische Schwerpunkt G einen Abstand A von weniger als 10 mm, insbesondere einen Abstand A von weniger als 5 mm aufweisen.

6. Plasma-Vorrichtung nach einem der vorhergehenden Ansprüche, wobei der Massenschwerpunkt M und der geometrische Schwerpunkt G identisch sind.

7. Plasma-Vorrichtung nach einem der vorhergehenden Ansprüche, wobei die Plasmaquelle (5) federbeaufschlagt ist und in einem Nichtgebrauchszustand der Plasma-Vorrichtung (1) über das Gehäuse (4) übersteht und in einem Gebrauchszustand zumindest teilweise in das Gehäuse (4) eingedrückt ist.

8. Plasma-Vorrichtung nach einem der Ansprüche 1 bis 7, wobei eine geometrische Längsachse (16) der Plasma-Vorrichtung (1) eine erste Kreisfläche (17) mit einem Radius R₁ ≤ 5,0 mm schneidet, wobei der Massenschwerpunkt M einen Mittelpunkt der ersten Kreisfläche (17) bildet.

9. Plasma-Vorrichtung nach einem der Ansprüche 1 bis 8, wobei eine geometrische Querachse (18) der Plasma-Vorrichtung (1) eine zweite Kreisfläche (19) mit einem Radius R₂ ≤ 5,0 mm schneidet, wobei der Massenschwerpunkt M einen Mittelpunkt der zweiten Kreisfläche (19) bildet.

10. Plasma-Vorrichtung nach einem der Ansprüche 1 bis 9, wobei eine geometrische Hochachse (20) der Plasma-Vorrichtung (1) eine dritte Kreisfläche (21) mit einem Radius R₃ ≤ 5,0 mm schneidet, wobei der Massenschwerpunkt M einen Mittelpunkt der dritten Kreisfläche (21) bildet.

11. Plasma-Vorrichtung nach Anspruch 8, 9 oder 10, wobei die geometrische Längsachse (16), die geometrische Querachse (18) und die geometrische Hochachse (20) der Plasma-Vorrichtung (1) eine Kugel mit einem Radius R_{K} ≤ 5,0 mm schneiden, wobei der Massenschwerpunkt M einen Mittelpunkt der Kugel bildet.

12. Plasma-Vorrichtung nach einem der Ansprüche 8 bis 11, wobei die Plasma-Vorrichtung (1) um zumindest die geometrische Längsachse (16), die geometrische Querachse (18) oder die geometrische Hochachse (20) symmetrisch ausgebildet ist.

13. Plasma-Vorrichtung nach einem der vorhergehenden Ansprüche, wobei ein Schalter (12) zum Ein- und Ausschalten der Plasma-Vorrichtung (1) vorgesehen ist, wobei der Massenschwerpunkt M und der Schalter (12) versetzt zum geometrischen Schwerpunkt G angeordnet sind.

14. Plasma-Vorrichtung (1) zum Behandeln von Oberflächen (2), insbesondere bei Textilien (3), wobei die Plasma-Vorrichtung (1) ein längliches und einhändig bedienbares Gehäuse (4), mit einem Gehäuseoberteil (14) und einem Gehäuseunterteil (15), aufweist, in welchem eine Plasmaquelle (5) angeordnet ist, die eine in Längsrichtung (6) des Gehäuses (4) verlaufende Plateaufläche (7) mit zumindest einer Elektrode (8) aufweist, über die Plasma erzeugbar und auf die zu behandelnde Oberfläche (2) ausbringbar ist, wobei ein Massenschwerpunkt M der Plasma-Vorrichtung (1) und ein geometrischer Schwerpunkt G der Plasma-Vorrichtung (1) um einen Abstand A voneinander beabstandet sind, der Abstand A weniger als 20 mm beträgt und der Massenschwerpunkt M vom geometrischen Schwerpunkt G in Richtung des Gehäuseoberteils (14) beabstandet ist.

15. Plasma-Vorrichtung nach Anspruch 14, wobei die Plasmaquelle (5) federbeaufschlagt ist und in einem Nichtgebrauchszustand der Plasma-Vorrichtung (1) über das Gehäuse (4) übersteht und in einem Gebrauchszustand zumindest teilweise in das Gehäuse (4) eingedrückt ist.

## Claims

1. Plasma device (1) for treating surfaces (2) of fabrics (3), wherein the plasma device (1) has an elongate housing (4) which can be operated with one hand, in which a plasma source (5) is arranged, which has a plateau surface (7) running in the longitudinal direction (6) of the housing (4) with at least one electrode (8), by way of which the plasma can be produced and can be applied to the surface (2) to be treated, wherein a centre of gravity M of the plasma device (1) and a geometrical centre G of the plasma device (1) has a distance A of less than 20 mm.

2. Plasma device according to claim 1, wherein the plasma device has a cross-sectionally oval-shaped housing (4).

3. Plasma device according to claim 2, wherein the oval-shaped cross-section of the housing (4) is adjusted to the palm of a hand such that the housing (4) can be gripped in a form-fit manner by a hand of a user.

4. Plasma device according to one of the preceding claims, wherein a holding frame (13) for the plasma source (5) is arranged accordingly and/or is adjusted in respect of its weight.

5. Plasma device according to one of the preceding claims, wherein the centre of gravity M and the geometrical centre G has a distance A of less than 10 mm, in particular a distance A of less than 5 mm.

6. Plasma device according to one of the preceding claims, wherein the centre of gravity M and the geometrical centre G are identical.

7. Plasma device according to one of the preceding claims, wherein the plasma source (5) is spring-loaded and in a non-use state of the plasma device (1) projects beyond the housing (4) and in a use state is impressed at least partially into the housing (4).

8. Plasma device according to one of claims 1 to 7, wherein a geometrical longitudinal axis (16) of the plasma device (1) intersects a first circular surface (17) with a radius R₁ ≤ 5,0 mm, wherein the centre of gravity M forms a centre point of the first circular surface (17).

9. Plasma device according to one of claims 1 to 8, wherein a geometrical transverse axis (18) of the plasma device (1) intersects a second circular surface (19) with a radius R₂ ≤ 5.0 mm, wherein the centre of gravity M forms a centre point of the second circular surface (19).

10. Plasma device according to one of claims 1 to 9, wherein a geometrical vertical axis (20) of the plasma device (1) intersects a third circular surface (21) with a radius R₃ ≤ 5.0 mm, wherein the centre of gravity M forms a centre point of the third circular surface (21).

11. Plasma device according to claim 8, 9, 10, wherein the geometrical longitudinal axis (16), the geometrical transverse axis (18) and the geometrical vertical axis (20) of the plasma device (1) intersect a ball with a radius R_{K} < 5.0 mm, wherein the centre of gravity M forms a centre point of the ball.

12. Plasma device according to one of claims 8 to 11, wherein the plasma device (1) is embodied symmetrically about at least the geometrical longitudinal axis (16), the geometrical transverse axis (18) or the geometrical vertical axis (20).

13. Plasma device according to one of the preceding claims, wherein a switch (12) is provided for switching the plasma device (1) on and off, wherein the centre of gravity M and the switch (12) are arranged offset with respect to the geometrical centre G.

14. Plasma device (1) for treating surfaces (2) in particular of fabrics (3), wherein the plasma device (1) has an elongate housing (4) which can be operated with one hand, having a housing upper part (14) and a housing lower part (15), in which a plasma source (5) is arranged, which has a plateau surface (7) running in the longitudinal direction (6) of the housing (4) with at least one electrode (8), by way of which the plasma can be produced and can be applied to the surface (2) to be treated, wherein a centre of gravity M of the plasma device (1) and a geometrical centre G of the plasma device (1) are distanced from one another by a distance A, the distance A is less than 20 mm and the centre of gravity M is distanced from the geometrical centre G in the direction of the housing upper part (14).

15. Plasma device according to claim 14, wherein the plasma source (5) is spring-loaded and in a non-use state the plasma device (1) projects beyond the housing (4) and in a use state is impressed at least partially into the housing (4).

## Revendications

1. Dispositif à plasma (1) pour le traitement de surfaces (2) en ce qui concerne des textiles (3), le dispositif à plasma (1) comprenant un boîtier (4) allongé et pouvant être utilisé à une seule main, dans lequel est disposée une source de plasma (5), laquelle comprend une surface de plateau (7) s'étendant dans la direction longitudinale (6) du boîtier (4) avec au moins une électrode (8), par l'intermédiaire de laquelle du plasma peut être produit et distribué sur la surface (2) à traiter, dans lequel un centre de masse M du dispositif à plasma (1) et un centre géométrique G du dispositif à plasma (1) sont espacés d'une distance A inférieure à 20 mm.

2. Dispositif à plasma selon la revendication 1, le dispositif à plasma comprenant un boîtier (4) de section transversale ovale.

3. Dispositif à plasma selon la revendication 2, dans lequel la section transversale ovale du boîtier (4) est adaptée à la paume d'une main de telle sorte que le boîtier (4) peut être enserré par une main d'une utilisatrice ou d'un utilisateur par complémentarité de forme.

4. Dispositif à plasma selon l'une des revendications précédentes, dans lequel un cadre de support (13) pour la source de plasma (5) est disposé de manière correspondante et/ou adapté en fonction de son poids.

5. Dispositif à plasma selon l'une des revendications précédentes, dans lequel le centre de masse M et le centre géométrique G sont espacés d'une distance A inférieure à 10 mm, en particulier d'une distance A inférieure à 5 mm.

6. Dispositif à plasma selon l'une des revendications précédentes, dans lequel le centre de masse M et le centre géométrique G sont identiques.

7. Dispositif à plasma selon l'une des revendications précédentes, dans lequel la source de plasma (5) est sollicitée par ressort et, dans un état de non-utilisation du dispositif à plasma (1), surmonte le boîtier (4) et, dans un état d'utilisation, est enfoncée au moins en partie dans le boîtier (4).

8. Dispositif à plasma selon l'une des revendications 1 à 7, dans lequel un axe longitudinal géométrique (16) du dispositif à plasma (1) coupe une première surface circulaire (17) avec un rayon R₁ ≤ 5,0 mm, dans lequel le centre de masse M forme un centre de la première surface circulaire (17).

9. Dispositif à plasma selon l'une des revendications 1 à 8, dans lequel un axe transversal géométrique (18) du dispositif à plasma (1) coupe une deuxième surface circulaire (19) avec un rayon R₂ ≤ 5,0 mm, dans lequel le centre de masse M forme un centre de la deuxième surface circulaire (19).

10. Dispositif à plasma selon l'une des revendications 1 à 9, dans lequel un axe vertical géométrique (20) du dispositif à plasma (1) coupe une troisième surface circulaire (21) avec un rayon R₃ ≤ 5,0 mm, dans lequel le centre de masse M forme un centre de la troisième surface circulaire (21).

11. Dispositif à plasma selon la revendication 8, la revendication 9 ou la revendication 10, dans lequel l'axe longitudinal géométrique (16), l'axe transversal géométrique (18) et l'axe vertical géométrique (20) du dispositif à plasma (1) coupent une sphère avec un rayon R_{K} ≤ 5,0 mm, dans lequel le centre de masse M forme un centre de la sphère.

12. Dispositif à plasma selon l'une des revendications 8 à 11, le dispositif à plasma (1) étant conçu symétriquement autour d'au moins l'axe longitudinal géométrique (16), l'axe transversal géométrique (18) ou l'axe vertical géométrique (20).

13. Dispositif à plasma selon l'une des revendications précédentes, dans lequel un commutateur (12) est prévu pour l'allumage et l'extinction du dispositif à plasma (1), dans lequel le centre de masse M et le commutateur (12) sont disposés de manière décalée par rapport au centre géométrique G.

14. Dispositif à plasma (1) pour le traitement de surfaces (2), en particulier en ce qui concerne des textiles (3), le dispositif à plasma (1) comprenant un boîtier (4) allongé et pouvant être utilisé à une seule main, avec une partie supérieure de boîtier (14) et une partie inférieure de boîtier (15), dans lequel est disposée une source de plasma (5), laquelle comprend une surface de plateau (7) s'étendant dans la direction longitudinale (6) du boîtier (4) avec au moins une électrode (8), par l'intermédiaire de laquelle du plasma peut être produit et distribué sur la surface (2) à traiter, dans lequel un centre de masse M du dispositif à plasma (1) et un centre géométrique G du dispositif à plasma (1) sont espacés l'un de l'autre d'une distance A, la distance A est inférieure à 20 mm et le centre de masse M est espacé du centre géométrique G dans la direction de la partie supérieure de boîtier (14).

15. Dispositif à plasma selon la revendication 14, dans lequel la source de plasma (5) est sollicitée par ressort et, dans un état de non-utilisation du dispositif à plasma (1), surmonte le boîtier (4) et, dans un état d'utilisation, est enfoncée au moins en partie dans le boîtier (4).
